# EUROPÄISCHE PATENTSCHRIFT

(11) **EP 0 427 246 B1**
(45) Veröffentlichungstag und Bekanntmachung des Hinweises auf die Patenterteilung: **09.08.1995**
(21) Anmeldenummer: 90121339.7
(22) Anmeldetag: 08.11.1990
(51) Int. Cl.: A61K 31/19, A61K 31/385

(54) **Arzneimittel, welche als Wirkstoff Schwefel enthaltende Carbonsäuren enthalten sowie deren Verwendung zur Bekämpfung von Retroviren**
Pharmaceutical compositions comprising sulfur containing carboxylic acids as the active agent as well as their use for combatting retroviruses
Compositions pharmaceutiques contenant comme principe actif des acides carboxyliques soufrés ainsi que leur utilisation dans la lutte contre les rétrovirus

(30) Priorität: 09.11.1989 DE 3937325; 16.05.1990 DE 4015728
(43) Veröffentlichungstag der Anmeldung: 15.05.1991
(73) Patentinhaber: ASTA Medica Aktiengesellschaft, D-01277 Dresden (DE)
(72) Erfinder: Kalden, Joachim, Prof. Dr., W-8520 Erlangen (DE); Fleckenstein, Bernhard, Prof. Dr., W-8551 Wiesenthau (DE); Baur, Andreas, Dr., W-8520 Erlangen (DE); Harrer, Thomas, Dr., W-8520 Erlangen (DE)

(56) Entgegenhaltungen:
- EP-A- 0 080 879
- EP-A- 0 318 891
- EP-A- 0 382 066
- EP-A- 0 427 247
- FR-A- 2 110 465
- US-A- 4 100 150
- STN FILE SERVER, File CA & CHEMICAL ABSTRACTS, Band CHEMICAL ABSTRACTS, Band87, Nr. 12, Zusammenfassung Nr. 090686, Columbus, Ohio, US; A. KOSHIRO et al.:"Studies on the incompatibility of parenteral solutions. (V). Incompatibilityof two kinds of polygelatin preparations", & KYUSHUYAKUGAKKAI KAIHO (KYYKBN); 76; vol. 30; pp. 57-66
- STN FILE SERVER, File CA & CHEMICAL ABSTRACTS, Band 12, Nr. 8, Zusammenfassung Nr. 062675, Columbus, Ohio, US; & JP-A-89 203 336 (KOKAI TOKYO KOHO) 16-08-1989
- PATENT ABSTRACTS OF JAPAN, Band 10, Nr. 31 (C-327)[2088], 6. Februar 1986;& JP-A-60 184 011 (ITARU YAMAMOTO) 19-09-1985
- DIALOG INFORMATION SERVICES, File 155 AN-85092554; V.V. IVANOVA et al.: "Effectof a number of preparations on the function of the immune system in experimental influenza in mice"", & VOPR VIRUSOL, Sept-Oct 1984, 29(5), p. 530-64, 29(5),p.530-5
- DIALOG INFORMATION SERVICES, FILE EMBASE, no. 83018751; H. THALER: "Drugtherapy of liver diseases in the eighties", & LEBER MAGEN DARM (GERMANY, WEST), 1982, 12/4 (171-174)
- DIALOG INFORMATION SYSTEM, & EMBASE, Nr. 84114649; A. DURST et al.: "Treatment of liver diseases: Advances in 1983", & THERAPIEWOCHE (GERMANY, WEST), 1984, 34/15 (2242-2258)

## Beschreibung

Die Erfindung betrifft spezifische Arzneimittel und Verfahren zur deren Herstellung, wobei diese Arzneimittel Wirkstoffe gemäß den Patentansprüchen 1, 2 und 3 enthalten. Diese Arzneimittel sind beispielsweise zur Bekämpfung von Retroviren (zum Beispiel HIV-Viren) beziehungsweise von Erkrankungen, die durch Retroviren hervorgerufen werden geeignet.

Im einzelnen betrifft die Erfindung folgende Arzneimittel und Verfahren zu deren Herstellung:

Ein Arzneimittel enthaltend mindestens eine Verbindung der allgemeinen Formel I, 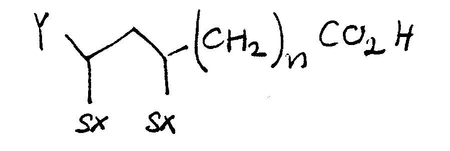
wobei X ein Wasserstoffatom ist, oder beide X eine einfache Bindung zwischen den beiden Schwefelatomen bedeuten; Y Wasserstoff oder C₁-C₆-Alkyl ist und n eine Zahl von 1 bis 10 darstellt oder deren therapeutisch verwertbaren Salze, zur Bekämpfung von HIV-Viren und von HIV-Viren verursachte Erkrankungen, wobei zwischen 400 mg - 6 g an Verbindung der allgemeinen Formel I in der Arzneimittelformulierung enthalten sind.

Die entsprechenden Arzneimittel können auch im Gemisch mit anderen pharmakologisch aktiven Stoffen verwendet werden. Insbesondere können die Wirkstoffe der Formel I auch mit anderen gegen Retroviren insbesondere HIV wirksamen Mittel kombiniert werden, beispielsweise mit Didesoxyinosin, Didesoxycytidin, insbesondere aber mit α-Interferon und/oder Azidothymidin (AZT).

Die erfindungsgemäßen Arzneimittel können jeweils als Wirkstoff eine einzige Verbindung der Formel I oder aber auch 2 und mehr Verbindungen der Formel I enthalten. Im letzteren Fall beziehen sich die angegebenen Dosierungen stets auf die Summe der Wirkstoffe gemäß der Formel I, das heißt, die Wirkkomponente I oder Komponente a. Dasselbe gilt ebenfalls bei Kombinationen, beispielsweise Kombinationen mit anderen antiretroviral-wirksamen Substanzen (Komponente b). Auch hier können als Komponente b nur eine, aber auch 2 und mehr (vorzugsweise 2) antiretroviral wirksame Substanzen verwendet werden, wobei im letzteren Falle auch hier die hierfür angegebenen Dosierungen stets für die Summe der jeweils vorliegenden antiretroviralwirksamen Stoffe gelten.
Der Ausdruck "Dosierungseinheit" bezieht sich stets auf eine Einzeldosis, die pro Tag auch mehrmals verabreicht werden kann.
Falls die Dosis in Form von Enzymeinheiten angegeben wird, handelt es sich um die Dosis, die für einen ganzen Tag gilt, wobei eine solche Dosis auf einmal, vorzugsweise jedoch verteilt über einen Tag (zum Beispiel in Form von Infusion) gegeben wird.
Die Dosis-Angabe in Enzymeinheiten gilt insbesondere für Kombinationen mit α-Interferon.

Weiterhin betrifft die Erfindung die Verwendung von Verbindungen der Formel 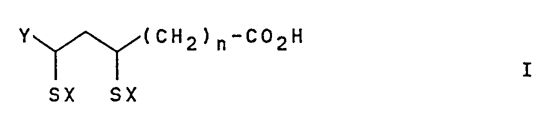
worin X ein Wasserstoffatom ist oder beide X eine einfache Bindung zwischen den beiden Schwefelatomen bedeuten; Y Wasserstoff oder C₁-C₆-Alkyl ist und n eine Zahl von 1 bis 10 darstellt, beziehungsweise deren therapeutisch verwendbaren Salzen zur Bekämpfung von Retroviren, insbesondere des Human Immundefiziusvirus (HIV) sowie Mittel zur Behandlung von Erkrankungen, die durch Retroviren verursacht werden beziehungsweise sind, welche Verbindungen der Formel I oder deren therapeutisch verwertbare Salze als Wirkstoffe enthalten.
Solche Arzneimittel können übliche pharmazeutische Träger-, Hilfs- und/oder Verdünnungsmittel enthalten. Die Erfindung betrifft daher auch ein Verfahren zur Formulierung von Wirkstoffen der Formel I mit üblichen pharmazeutischen Träger-, Hilfs- und/oder Verdünnungsmitteln zu einem Arzneimittel für die Behandlung von durch Retroviren verursachten Erkrankungen.

Die Mittel zur Bekämpfung von Retroviren enthalten die Wirkstoffe der Formel I beispielsweise in einer solchen Menge, daß bei ein- oder mehrmaliger Applikation täglich im Körper ein Wirkspiegel zwischen 3,5 und 200 mg/kg Körpergewicht vorliegt.

Die Erfindung betrifft darüberhinaus auch ein Erzeugnis zur Bekämpfung von Retroviren, welches dadurch gekennzeichnet ist, daß es neben den üblichen pharmazeutischen Träger- und/oder Verdünnungsbeziehungsweise Hilfsstoffen
a) eine Verbindung der Formel 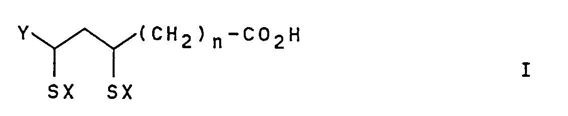 worin X ein Wasserstoffatom ist oder beide X eine einfache Bindung zwischen den beiden Schwefelatomen bedeuten; Y Wasserstoff oder C₁-C₆-Alkyl ist und n eine Zahl von 1 bis 10 darstellt und
b) eine andere antiretroviralwirksame Substanz
oder jeweils ein physiologisch unbedenkliches Salz beider Wirkstoffe, in einer eine synergistische Wirkung erzeugenden Menge enthält und in einer Form vorliegt, die sowohl eine gemeinsame als auch eine getrennte therapeutische Anwendung beider Wirkstoffe gestattet.

Die andere gegen Retroviren wirksame Substanz kann zum Beispiel sein:
Azidothymidin (AZT), Didesoxyinosin (DDI), α-Interferon oder Didesoxycytidin (DDC).

Das vorstehend erwähnte Erzeugnis ist beispielsweise dadurch gekennzeichnet, daß in der Kombination auf ein Gewichtsteil einer Verbindung der Formel I (Wirkkomponente I beziehungsweise Komponente a) jeweils 0,01 bis 100 Gewichtsteile der anderen Komponente gemäß b) oder auf 1 mg Wirkkomponente I (Komponente a) jeweils 20 - 200 000 Enzymeinheiten der anderen Komponente gemäß b) kommen, beziehungsweise daß in der Dosierungseinheit die Kombination 50 mg bis 6 g, vorzugsweise 200 mg bis 2 g Wirkkomponente I und 10 bis 300 mg, vorzugsweise 50 bis 200 mg beziehungsweise 5 x 10⁵ Enzymeinheiten (Units) bis 8 x 10⁶ Enzymeinheiten, vorzugsweise 1 x 10⁶ bis 4 x 10⁶ Enzymeinheiten der anderen Komponente gemäß b) enthält.

Zusätzlich kann ein solches Mittel/Erzeugnis auch Vitamine, vorzugsweise Vitamine B₁ und/oder E enthalten.

Ebenfalls betrifft die Erfindung ein Verfahren zur Herstellung eines wie vorstehend beschriebenen Erzeugnisses, welches dadurch gekennzeichnet ist, daß man 1 Gewichtsteil Wirkkomponente I und 0,01 bis 100 Gewichtsteile beziehungsweise 20 bis 200 000 Enzymeinheiten der anderen Komponente gemäß b), wobei die Wirkstoffe auch in Form ihrer Salze vorliegen können, zusammen mit üblichen Träger- und/oder Verdünnungs- beziehungsweise Hilfsstoffen sowie gegebenenfalls unter Zusatz von Vitaminen zu Erzeugnissen verarbeitet, welche in der Dosierungseinheit 50 mg bis 6 g Wirkkomponente I und 10 bis 300 mg beziehungsweise 5 x 10⁵ bis 8 x 10⁶ Enzymeinheiten der anderen Komponente gemäß b) enthalten.

Die Verbindungen der Formel I einschließlich der α-Liponsäure und Dihydroliponsäure können auch in Form ihrer optisch isomeren (R(+)- und S-(-)-Form, üblicherweise liegen die Verbindungen I als Racemate vor) beziehungsweise diastereomeren Formen für die Herstellung pharmazeutischer Zusammensetzungen und Zubereitungen sowie die angegebene Anwendung verwendet werden. Vorzugsweise kommen als Verbindungen der Formel I die α-Liponsäure und die Dihydroliponsäure (Racemate sowie die entsprechenden Enantiomeren) in Frage.

α-Liponsäure ist in Form des Racemats (Thioctsäure®) in Pflanzen und Tieren weit verbreitet; sie wirkt in vielen enzymatischen Reaktionen als Coenzym, stellt einen Wachstumsfaktor für manche Bakterien und Protozoen dar und wird bei Knollenblätterpilzvergiftungen eingesetzt. Weiterhin weist das α-Liponsäure-Racemat antiphlogistische, antinociceptive (analgetische) sowie zytoprotektive Eigenschaften auf. Thiocsäure ist als Arzneimittel für folgende Indikationen auf dem Markt:
Fettleber und Fettzirrhose, besonders durch Alkohol chronische Lebererkrankung,
durch Pilzvergiftung verursachte Leberschädigung, diabetische Neuropathie,
alkoholische Neuropathie.

Dihydroliponsäure ist die 6,8-Dimercapto-octansäure. Aus Tieruntersuchungen ist bekannt, daß Dihydroliponsäure Schlangengift inaktiviert. Diese Untersuchungen erfolgten beispielsweise an Ratten und Mäusen, wobei Lösungen in Wasser oder physiologische Kochsalzlösung verwendet wurden, die das Schlangengift und Dihydroliponsäure enthielten.

In den bisher bekannten Zubereitungen sind α-Liponsaure und Dihydroliponsäure in relativ niedrigen Mengen vorhanden.
Die erfindungsgemäßen Arzneimittel mit höheren Mengen an α-Liponsäure beziehungsweise Dihydroliponsäure sind neu; es war außerdem nicht naheliegend, daß dies Wirkstoffe in höherer Dosierung vorteilhaftere pharmazeutische Wirkungen zeigen, beispielsweise bei der Behandlung von Aids.

Die Herstellung der erfindungsgemäßen Arzneimittel erfolgt in bekannter Weise, wobei die bekannten und üblichen pharmazeutischen Hilfsstoffe sowie sonstige übliche Träger- und Verdünnungsmittel verwendet werden können. Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Enzyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2 (1961), Seite 72 ff.; Dr. H.P. Fiedler, Lexikon der Hilfsstoffe für Pharmazie, Kosmetik und angrenzende Gebiete, Cantor KG, Aulendorf in Württemberg (1989).

Die pharmazeutische und galenische Handhabung der Verbindungen der Formel I erfolgt nach den üblichen Standardmethoden. Beispielsweise werden die Wirkstoffe und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 50° C, vorzugsweise 20 bis 40° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation der Arzneimittel kann auf die Haut oder Schleimhaut oder in das Körperinnere erfolgen, beispielsweise oral, enteral, pulmonal, nasal, lingual, intravenös, intraarteriell, intrakardial, intramuskulär, intraperitoneal, intracutan, subcutan.

Bei den parenteralen Zubereitungsformen handelt es sich insbesondere um sterile beziehungsweise sterilisierte Erzeugnisse.

Die Verbindung der Formel I können auch in Form ihrer therapeutisch verwendbaren Salze eingesetzt werden. Die Herstellung solcher Salze erfolgt in hierfür bekannter Weise.
Als Salzbildner kommen zum Beispiel übliche Basen beziehungsweise Kationen in Frage, die in der Salzform physiologisch verträglich sind. Beispiele hierfür sind:
Alkali- oder Erdalkalimetalle, Ammoniumhydroxid, basiche Aminosäuren wie Arginin und Lysin, Amine der Formel NR₁R₂R₃ worin die Reste R₁, R₂ und R₃ gleich oder verschieden sind und Wasserstoff, C₁-C₄-Alkyl oder C₁-C₄-Oxyalkyl bedeuten wie Mono- und Diethanolamin, 1-Amino-2-propanol, 3-Amino-1-propanol; Alkylendiamine mit einer Alkylenkette aus 2 bis 6-C-Atomen wie Ethylendiamin oder Hexamethylentetramin, gesättigte cyclische Aminoverbindungen mit 4 - 6 Ringkohlenstoffatomen wie Piperidin, Piperazin, Pyrrolidin, Morpholin; N-Methylglucamin, Dreatin, Trometamol.

Falls die Verbindungen der Formel I in Form ihrer Salze verwendet werden, kann der Salzbildner auch im Überschuß eingesetzt werden, das heißt in einer höheren Menge als äquimolar.

Beispiele für die Träger- und Hilfsstoffe sind Gelatine, natürliche Zucker wie Rohrzucker oder Milchzucker, Lecithin, Pektin, Stärke (zum Beispiel Maisstärke oder Amylose), Cyclodextrine und Cyclodextrinderivate, Dextran, Polyvinylpyrrolidon, Polyvinylacetat, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rizinusöl,
Olivenöl, Sesamöl, Baumwollsaatöl, Maisöl, Weizenkeimöl, Sonnenblumensamenöl, Kabeljau-Leberöl, jeweils auch hydriert); Glycerinester und Polyglycerinester aus gesättigten Fettsäuren C₁₂H₂₄O₂ bis C₁₈H₃₆O₂ und deren Gemische, wobei die Glycerin-Hydroxygruppen vollständig oder auch nur teilweise verestert sind (zum Beispiel Mono-, Di- und Triglyceride); pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Harnstoff, Benzylbenzoat, Dioxolane, Glyzerinformale, Tetrahydrofurfurylalkohol, Polyglykolether mit C₁-C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit® RL); Polyvinylacetat; Fette, Öle,
Wachse, Fettalkohole; Hydroxypropylmethylcellulosephthalat oder -acetatsuccinat; Cellulose-acetatphthalat; Stärke-acetatphthalat sowie Polyvinylacetatphthalat; Carboxymethylcellulose; Methylcellulosephthalat, Methylcellulosesuccinat, -phthalatsuccinat sowie Methylcellulose-phthalsäurehalbester; Zein; Ethylcellulose sowie Ethylcellulosesuccinat; Schellack, Gluten; Ethylcarboxyethylcellulose; Ethacrylat-Maleinsäureanhydrid-Copolymer; Maleinsäurenanhydrid-Vinylmethylether-Copolymer; Styrol-Maleinsäure-Copolymerisate; 2-Ethyl-hexyl-acrylatmaleinsäureanhydrid; Crotonsäure-Vinylacetat-Copolymer; Glutaminsäure/Glutaminsäureester-Copolymer; Carboxymethylethyl-celluloseglycerinmonooctanoat; Celluloseacetatsuccinat; Polyarginin.

Als Plastifizieruhgsmittel für Hüllstoffe können in Frage kommen:
Citronen- und Weinsäureester (Acetyltriethylcitrat, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), Di-(2-Methoxy- oder 2-ethoxyethyl)-phthalat, Ethylphthalylglycolat, Butylphthalylethylglycolat und Butylglycolat; Alkohole (Propylenglycol, Polyethylenglycol verschiedener Kettenlängen), Adipate (Diethyl-adipat, Di-(2-Methoxy-oder 2-ethoxyethyl)-adipat); Benzophenon; Diethyl- und Dibutylsebacat, Dibutylsuccinat, Dibutyltartrat; Diethylenglycoldipropionat; Ethylenglykoldiacetat, -dibutyrat, -dipropionat; Tributylphosphat, Tributyrin; Polyethylenglykolsorbitanmonooleat (Polysorbate wie Polysorbat 80); Sorbitanmonooleat.

Zur Herstellung von Lösungen oder Suspensionen kommen beispielsweise Wasser oder physiologisch verträgliche organische Lösungsmittel in Frage, wie zum Beispiel Alkohole (Ethanol, Propanol, Isopropanol, 1,2-Propylenglykol, Polyglykole und deren Derivate, Fettalkohole, Partialester des Glycerins), Öle (zum Beispiel Erdnußöl, Olivenöl, Sesamöl, Mandelöl, Sonnenblumenöl, Sojabohnenöl, Ricinusöl, Rinderfußöl), Paraffine, Dimethylsulfoxid, Triglyceride und ähnliche.
Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Glycerol, Ringer's Lösung, isotonische Kochsalzlösung oder auch gehärtete Öle einschließlich synthetischer Mono-oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkyl phenolen oder Fettsäuren oder auch 1-Methyl-3-(2-hydroxyethyl)imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro 1 Mol Glycerid).
Beispiele für Oleotriglyceride sind Olivenöl, Erdnußöl, Rizinusöl, Sesamöl, Baumwollsaatöl, Maisöl. Siehe auch Dr. H. P. Fiedler "Lexikon der Hilfsstoffe für Pharmazie, Kosmetik und angrenzende Gebiete" 1971, Seite 191 - 195.

Für wässrige Injektions- und Trinklösungen werden als Stabilisatoren beziehungsweise Lösungsvermittler, insbesondere folgende Substanzen eingesetzt: niedere aliphatische ein- und mehrwertige Alkohole mit 2 - 4 C-Atomen wie Ethanol, n-Propanol, Glycerin, Polyethylenglykole mit Holgewichten zwischen 200 - 600 (zum Beispiel 1 bis 40 %ige wässrige Lösung), Diethylenglykolmonoethylether, 1,2-Propylenglykol, organische Amide, zum Beispiel Amide aliphatischer C₁-C₆-Carbonsäuren mit Ammoniak oder primären, sekundären oder tertiären C₁-C₄-Aminen oder C₁-C₄-Hydroxyaminen wie Harnstoff, Urethan, Acetamid, N-Methylacetamid, N,N-Diethylacetamid, N,N-Dimethylacetamid, niedere aliphatische Amine und Diamine mit 2-6 C-Atomen wie Ethylendiamin, Hydroxyethyltheophyllin, Trometamol (zum Beispiel als 0,1 bis 20 %ige wässrige Lösung), aliphatische Aminosäuren.
Bei den Aminosäuren handelt es sich zum Beispiel um Aminosäuren der folgenden Struktur: 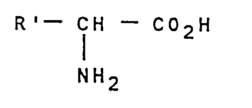
worin R' Wasserstoff, einen Phenylrest, einen Indolyl-(3)-methylrest, Imidazolyl-(4)-methylrest, eine C₁-C₁₀-Alkylgruppe oder eine C₁-C₁₀-Alkylgruppe, die durch eine Hydroxygruppe, eine Carboxygruppe, eine C₁-C₆-Alkoxygruppe, eine Mercaptogruppe, eine C₁-C₆-Alkylthiogruppe, eine Aminogruppe, eine Phenylgruppe, eine Hydroxyphenylgruppe, eine C₂-C₆-Alkanoylaminogruppe oder eine C₁-C₆-Alkoxycarbonylgruppe substituiert ist, bedeutet.

Beispielsweise kommen als Trink- beziehungsweise Injektionslösungen folgende Rezepturen in Frage:

| | |
|---|---|
| α-Liponsäure | 10 % |
| L-Lysin | 7,66 % |
| Ethylendiamin | 0,27 % |
| Wasser | 82,07 % |

| | |
|---|---|
| α-Liponsäure | 10 % |
| L-Lysin | 7,66 % |
| Trometamol | 1 % |
| Wasser | 81,34 % |

| | |
|---|---|
| Dihydroliponsäure | 1 % |
| Trometamol | 0,9 % |
| Ethylendiamin | 0,38 % |
| Wasser | 97,72 % |

| | |
|---|---|
| Dihydroliponsäure | 1 % |
| Trometamol | 1,5 % |
| 1,2-Propylenglykol | 20 % |
| Nikotinsäureamid | 10 % |
| Wasser | 67,5 % |

Darüberhinaus ist der Zusatz von Konservierungsmitteln, Stabilisatoren, Puffersubstanzen, Geschmackskorrigentien, Süßmitteln, Farbstoffen, Antioxydantien und Komplexbildnern und dergleichen möglich.
Als Komplexbildner kommen beispielsweise in Frage: Chelatbildner wie Ethylendiamino-tetraessigsäure, Nitrilotriessigsäure, Diethylentriaminpentaessigsäure sowie deren Salze.
Weiterhin kommen als Komplexbildner auch solche in Frage, die R- oder S-α-Liponsäure in einem Hohlraum einschließen. Beispiele hierfür sind Harnstoff, Thioharnstoff, Cyclodextrine, Amylose.
Gegebenenfalls ist zur Stabilisierung des Wirkstoffmoleküls mit physiologisch verträglichen Basen oder Puffern auf einen pH-Bereich von ca. 6 bis 9 einzustellen. Im allgemeinen wird ein möglichst neutral er bis schwach basischer (bis pH 8) pH-Wert bevorzugt.

Als Antioxydantien kommen beispielsweise Natriumsulfit, Natriumhydrogensulfit, Natriummetabisulfit, Ascorbinsäure, Ascorbylpalmitat, -myristat, -stearat, Gallussäure, Gallussäure-alkylester, Butylhydroxyanisol, Nordihydroguajaretsäure, Tocopherole sowie Synergisten (Stoffe, die Schwermetalle durch Komplexbildung binden, beispielsweise Lecithin, Ascorbinsäure, Phosphorsäure Ethylendiaminotetraessigsäure, Citrate, Tartrate) zur Anwendung. Der Zusatz der Synergisten steigert die antioxygene Wirkung der Antioxydantien erheblich.
Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid, Chlorhexidin und Formalinderivate in Betracht.

Die erfindungsgemäßen Verbindungen der allgemeinen Formel I besitzen insbesondere auch eine wachstumshemmende Wirkung gegen Retroviren, insbesondere das HumanImmundefizienzvirus (HIV, zum Beispiel HIV-1, HIV-2) und eine aktivierende und wachstumsfördernde Wirkung auf periphere mononukleäre Blutzellen. Beispielsweise verlangsamen, beziehungsweise hemmen die Verbindungen I die Vermehrung des Virus.

Als Retroviren, gegen die die erfindungsgemäßen Verbindungen/Erzeugnisse wirksam sind, kommen zum Beispiel in Frage: HIV-Viren, Oncornaviren, Spumaviren.

Beispielsweise besitzen die Verbindungen der Formel I eine gute, wachstumshemmende Wirkung auf HIV (Typ 1 und 2), welche sich in vitro beispielsweise durch folgende virologisch-zellbiologische Testverfahren zeigen läßt:
1. Plaque - Reduktionstest
2. CPE - Reduktionstest
3. Bestimmung der Reversen Transkriptase im Kulturüberstand
4. Bestimmung des p24 Antigens im Kulturüberstand
So wird beispielsweise bei einer einmaligen Gabe von 0,035 mg/ml Verbindung I (zum Beispiel alpha-Liponsäure, Racemat) die Anzahl der infektiösen Viren (zum Beispiel HIV-1), im Zellkulturüberstand von 100 % in der positiv Kontrolle auf 0 % gesenkt. Ein virusinhibierender Effekt kann in diesem Testverfahren bereits in sehr niedrigen Dosen, beispielsweise 0,001 mg/ml nachgewiesen werden.

Als allgemeiner Dosisbereich für die Wirkung (Versuch wie oben) kommt beispielsweise in Frage: 0,0035 - 0,091 mg/ml, insbesondere 0,035 - 0,070 mg/ml.

Für die in vitro Versuche wird der Wirkstoff der Formel I, zum Beispiel in Benzylalkohol als Lösungsmittel eingesetzt.

Für die in vitro Untersuchungen des Replikationsverhaltens von Retroviren, insbesondere HIV, können zum Beispiel folgende Substrate eingesetzt werden:
1. Virushaltiges RPMI 1640 Medium, zum Beispiel 1X Flüssig 041-01875 (synthetisches Kulturmedium von Gibeo gemäß Moore, Gerner und Franklin, H.A. (1967), J.A.M.A. 199; 519) mit einer Konzentration von 2 x 10³ - 1 x 10⁴ infektiösen Einheiten (PFU)/ml
2. Die Zellinien Jurkat Clone E6-1, Sup T1 und HeLa CT4.
   Zellinie Jurkat Klon E6-1
   Zelltyp: Humane T-Zelleukämie. Wachstumsmedium: RPMI 1640, 90 %; fetales Kälberserum, 10 %. Einfriermedium: Kulturmedium, 90 %; Dimethylsulfoxid, 10 %. Anteil lebender Zellen: 80 %. Wachstumscharakteristiken: Die Zellen werden alle 2 bis 3 Tage passagiert. Die Zellzahl ist zwischen 10⁵ und 10⁶ Zellen/ml zu halten. Morphologie: lymphozytär. Karyologie: nicht angegeben. Sterilität: Bakterien und Mykoplasmen negativ. Reverse Transkriptase: negativ. Spezielle Eigenschaften: Dieser Klon von Jurkat-FHCRC (Dr. Kendall Smith, Dartmouth) produziert große Mengen von Interleukin-2 (IL-2) nach geeigneter Stimulation. Die Zellen können induziert werden, Gamma-Interferon zu sezernieren und sind CD4⁺. Quelle: ATCC durch Dr. Arthur Weiss. Referenzen: Journal of Immunology, 133:123, 1984.
   Zellinie Sup-T1
   Zelltyp: Non-Hodgkin's T-Zelllymphom. Wachstums-medium: McCoy's 5A Medium, 85 %; fetales Kälberserum, 15 %. Die Zellen wachsen auf einem feeder layer (komplettes Medium mit 10 % normalen Humanserum and 0,5 % Agar). Einfrier-medium, 90 %; Dimethylsulfoxid, 10 %. Anteil lebender Zellen: 80 %. Wachstumseigenschaften: Die Zellen werden passagiert, wenn die Zellkonzentration größer als 5 x 10⁵/ml wird. Für die Passage wird die Kultur 1:10 bis 1:20 mit frischem Wachstumsmedium verdünnt. Morphologie: ausgereift lymphozytär. Karyologie: nicht angegeben. Sterilität: Bakterien und Mykoplasmen negativ. Reverse Transkriptase: negativ. Spezielle Eigenschaften: Die Zellen sind TdT positiv, CALLA negativ, DR negativ. Sie exprimieren pan T-Antigene, besitzen keinen Schaferythrozytenrezeptor und exprimieren hohe Spiegel von Oberflächen-CD4. Quelle: Dr. James Hoxie, Referenzen: Cancer Research 44 : 5657, 1984.
3) Zellinie HeLa T4⁺
   Zelltyp: Human epithelialartig. Wachstumsmedium: Kulturmedium bestehend aus verschiedenen Aminosäuren und Elektrolyten für die Kultivierung epithelialer Zellen (zum Beispiel Dulbecco's minimal essential medium, DME), 90 %; Serum von neugeborenen Kälbern, 10 %. Einfriermedium: Kulturmedium, 95 %; Glyzerin, 5 %, ohne Antibiotika. Anteil lebender Zellen, 80 %. Wachstumseigenschaften wie Ursprungslinie. Morphologie: änlich der Ursprungslinie. Karyologie: nicht angegeben. Sterilität: Bakterien und Mykoplasmen negativ. Reverse Transkriptase: negativ. Besondere Eigenschaften: vor dem retrovirus-vermittelten Gentransfer mit CD4cDNA exprimieren diese Zellen kein Oberflächen-CD4 und sind nicht empfindlich für die AIDS Virusinfektion. Nach der Transfektion erlauben CD4+-Zellen die Infektion durch AIDS-Virus und die Induktion von Synzytien. Quelle: Dr. Richard Axel. Referenzen: Cell 47 : 333, 1986.

Weiteres zur HIV Inhibition durch α-Liponsäure in vitro.

Die Hemmwirkung wird an einer mit HIV-1 permanent infizierten Zellinie gezeigt.

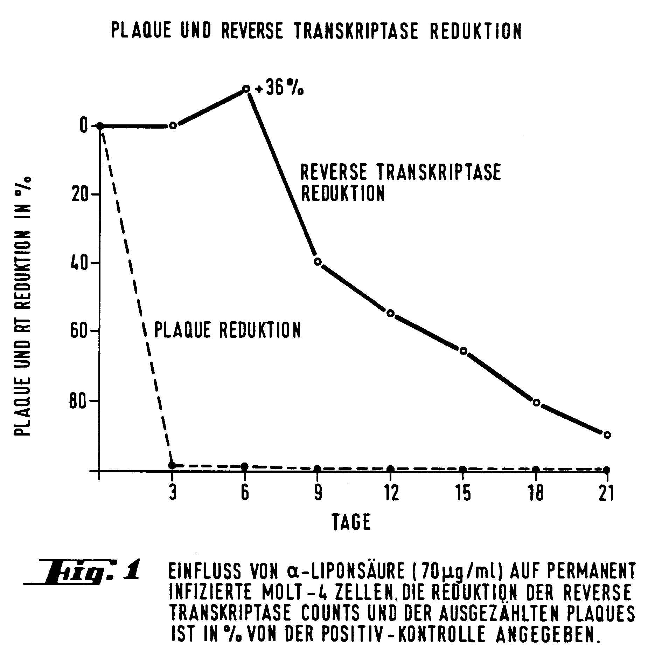

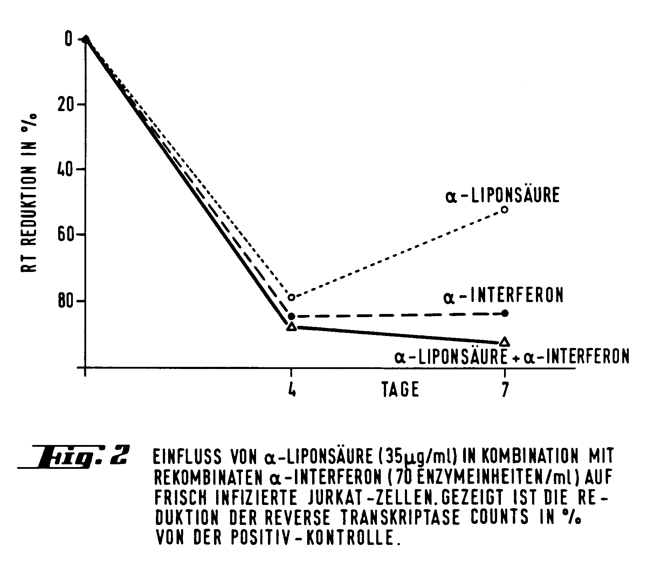

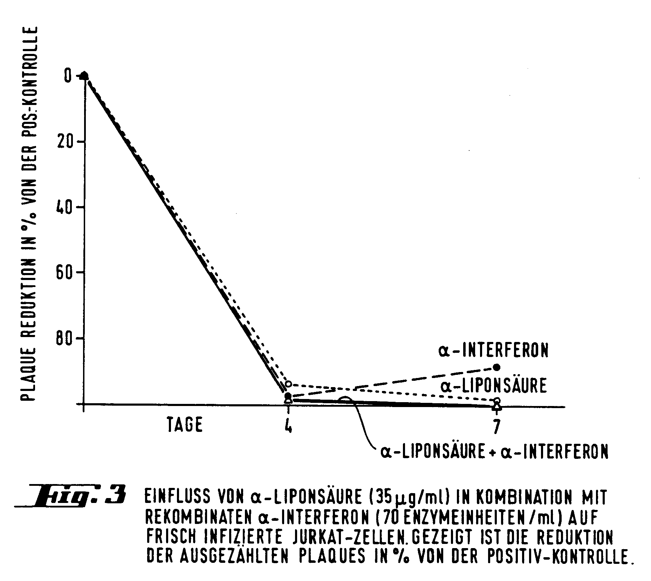

Herstellen einer permanent infizierten Zellinie mit HIV-1:
1. Negative (nicht infizierte) Zellen (zum Beispiel Molt 4) werden aus der Kulturflasche mit Kulturmedium RPMI 1640 entnommen und in einem geeigneten Gefäß herunterzentrifugiert.
   Als negative Zellen können grundsätzlich die Zellen aus jeder CD4-Rezeptor positiven T-Zellinie verwendet werden. Beispielsweise kommen die folgenden im Handel erhältlichen Zellinien in Frage: H9, Hut78, SupT1, Jurkat, Molt4. Molt4 ist beispielsweise eine Monoclonale T-Zellinie aus peripherem Blut eines Krebskranken. T-Zellinien leiten sich ab von menschlichen Tumoren, zum Beispiel von bestimmten Leukämieformen. Derartige Zellinien wachsen permanent solange sie in entsprechendem Nährmedium und CO₂-Konzentration in einem Brutschrank bei 37°C gehalten werden. Erworben werden können diese Linien von der sogenannten American Type Culture Collection (ATCC).
2. Diese Molt4 Zellen werden dann in hoch-virushaltigem Kulturmedium (RPMI 1640 + 10 % fötales Kälberserum) resuspendiert (aufgenommen), in eine Kulturflasche gegeben und ca. 12 Stunden im Brutschrank bei 37°C und 5%CO₂ inkubiert (kultiviert). Hierdurch wird ein Teil (je nach Konzentration des infektiösen Virus) der Zellen infiziert.
3. Nach 12 Stunden werden die Zellen erneut abzentrifugiert und in frischem Medium (RPMI 1640, nicht virushaltig) resuspendiert und im Brutschrank für ca. 4 Wochen kultiviert.
4. Dabei wird jede Woche einmal das Medium komplett gewechselt (abzentrifugiert, resuspendiert, usw.) und negative, nicht infizierte Zellen werden hinzugegeben.
5. Nach ca. 4 Wochen sind 80 - 100 % der Zellen infiziert = permanent infizierte Zellinie. Die Infizierung wird beispielsweise durch Immunfluoreszenz bestimmt. Die so infizierten Zellen produzieren HIV in einem Titerbereich von etwa 10⁵ - 10⁶ infektiösen Einheiten (PFU). Die so permanent infizierte Tumorzellinie (Molt4) wird drei Wochen lang mit 70 »g α-Liponsäure/ml behandelt. Hierbei wurde alle drei Tage Kulturmedium und α-Liponsäure erneuert und die Aktivität des Virus im Reverse Transkriptase sowie Plaque Test bestimmt. Der Reverse Transkriptase Test ermittelt die relative Menge an gebildetem Virus (auch an infektionsdefekten Partikeln) während der Plaque Test nur die infektiösen Viruseinheiten zu bestimmen vermag.
   Der gewählte Konzentrationsbereich von 70 »g/ml liegt ca. um das 10 - 20 fache über Plasmaspiegeln, die bei normaler oraler, nebenwirkungsfreier Dosierung gemessen wurden. Die Proliferationsrate beziehungswiese Letalität von peripheren Blutlymphozyten ist bei dieser Dosierung nicht wesentlich beeinflußt.
   Wie die Kurven gemäß Figur 1 zeigen, sinkt die Zahl der Infektiösen Viruseinheiten bereits nach 3 Tagen auf annähernd 0, was einer nahezu 100%igen Reduktion entspricht. Die Zahl der produzierten Viruseinheiten sinkt erst ab Tag 6 ab und erreicht nach drei Wochen eine Reduktion von 90 %. Diese Ergebnisse demonstrieren die starke antivirale Potenz von α-Liponsäure. Als zweites wichtiges Ergebnis bleibt festzuhalten, daß sich im Konzentrationsbereich von 70 »g/ml nach drei Wochen keine Toleranzentwicklung gezeigt oder abgezeichnet hat. Dies ist insbesondere für eine Langzeitbehandlung von infizierten Personen von Bedeutung.
   Will man die in vitro Wirkung von α-Liponsäure mit anderen Mitteln vergleichen, die bereits mit Erfolg in der Therapie der AIDS Erkrankung eingesetzt werden, bietet sich das alpha Interferon an. Die antivirale Wirkung dieser Substanz wird auf posttranslationeller Ebene vermutet, das heißt eine hemmende Wirkung auf den sogenannten Budding- oder Ausschleusprozeß der Viren wird diskutiert. Ähnlich wie α-Liponsäure wirkt alpha Interferon daher auf die bereits infizierte Zelle. Um beide Verbindungen zu vergleichen, wurden frisch gesplittete Jurkat Zellen mit HIV infiziert (8 x 10³ PFU) und anschließend rekombinantes alpha Interferon (rIF) (70 Einheiten/ml ) beziehungsweise 35 ng/ml α-Liponsäure hinzupipettiert. Der Versuch wurde nach 7 Tagen terminiert, um insbesondere das Wachstum der ersten Virusgeneration zu beurteilen. Beide Substanzen zeigten eine vergleichbare Hemmung der viralen Replikation in den infizierten, nicht vorbehandelten Zellen (Figur 2 und 3). Während allerdings rIF eine stärkere inhibierende Wirkung im Reverse Transkriptase Test zeigt (mißt die relative Menge produzierter Virionen, Figur 2), ist die Hemmung im Plaque Test (gibt die genau Anzahl infektiöser Virionen an, Figur 3) für α-Liponsäure deutlicher. Die Kombination beider Substanzen zeigt eine additive Wirkung.
   Die erfindungsgemäßen Verbindungen können bei der Behandlung von Aids insbesondere auch mit AZT kombiniert werden, da AZT einen unterschiedlichen Wirkmechanismus besitzt. AZT hemmt die Reverse Transkriptase und wirkt somit überwiegend auf nicht infizierte Zellen. Ist eine Zelle einmal infiziert, kann AZT im Gegensatz zu α-Liponsäure das Viruswachstum jedoch nicht mehr hemmen.

Herstellen von frisch infizierten Zellen
1. Wie bei der Herstellung einer mit HIV-1 permanent infizierten Zellinie beschrieben einschließlich Punkt 2.
2. Zellen werden in frischem Medium aufgenommen und können jetzt als frisch infizierte Zellen bezeichnet werden. Im Unterschied zu permanent infizierten Zellen sind weit weniger Zellen Virusträger, dafür jedoch synchron behandelt, das heißt die erste und zweite Virusgeneration wird relativ gleichzeitig etwa am 3. und 7. Tag in den Kulturüberstand abgegeben. Diese Virusgenerationen können an diesen Tagen quantifiziert (Plaque- und Reverse-Transkriptase-Test) und verglichen werden. Man spricht in diesem Zusammenhang auch von einer "single hit Kinetik".
3. α-Liponsäure wird hinzupipettiert.
4. Zellen werden für 7 Tage im Brutschrank kultiviert.
5. Am Tag 4 und 7 werden Proben aus der Kultur entnommen für Plaque- und RT-Assay.

### Hemmung der HIV Replikation durch α-Liponsäure in vivo

### Methodik

- Bestimmung des Plasma p24 Antigenspiegels mittels eines kommerziellen ELISA p24 ist die Bezeichnung für ein Strukturprotein des HIV-Virus ELISA (Abkürzung für enzyme linked immuno sorbent assay) ist eine häufig in der Virologie angewendete Testtechnik zur Bestimmung von Proteinen, Antigenen usw.
- Virusisolierung mit Kokultivierung: Patientenlymphozyten werden mit Spenderlymphozyten kokultiviert. Das von diesen Zellen produzierte Virus (p24) wird nach 21 Tagen in einem ELISA gemessen (Standardmethode).
- Virusisolierung ohne Kokultivierung: Die Produktion von Virus (p24) aus 1 x 10⁶ Patientenlymphozyten wird nach 11 Tagen mit einem ELISA quantitativ gemessen. Bei dieser standardisierten Methode werden keine Spenderlymphozyten zugegeben. Die Virusproduktion kann in pg*p24/ml/200000 Zellen angegeben werden. (Neu entwickelte Methode).
- Zelltitration: Ausgehend von einer absteigenden Zahl von Patientenlymphozyten (z.B. 10⁶,10⁵,10⁴) wird eine Virusisolierung durchgeführt.
- Bestimmung des Plasmatiters, welcher die Anzahl der infektiösen Viren im Plasma angibt.

* pg = Pico-Gramm

### Ergebnisse

Die folgenden Ergebnisse wurden an 4 Patienten im Walter-Reed Stadien 6 erhalten. Applikation der α-Liponsäure erfolgte durch Infusion einer α-Liponsäure-Lösung folgender Zusammensetzung: 10 ml wässrige Lösung enthalten 250 mg α-Liponsäure in Form des Äthylendiaminsalzes (= 323 mg Salz) sowie 1 g 1,2-Propylenglykol und 100 mg Benzylalkohol.

Walter-Reed-Stadien: Einteilung von HIV-infizierten Patienten nach dem Grad ihres klinischen Erscheinungsbildes. Bei Stadium 0 ist kein HIV-Virus nachweisbar. Stadium 6 bedeutet finales Aids-Stadium.

### Patient 1 (K.M., 64 kg; Walter-Reed-Stadium 6)

Therapie-Phase 58 Tage, wobei jedoch 2 x 36 Stunden wegen Urlaubs unterbrochen wurden. Die Gesamtdosis betrug 258,3 g, was einer durchschnittlichen Tagesdosis von 4,7 g/Tag entspricht. An 19 Tagen wurden Dosen von 3 g bis weniger als 6 g, an 18 Tagen Dosen von 6-8 g/Tag verabreicht.
Die Infusion von α-Liponsäure erfolgte über einen zentralen Venenkatheter über 24 Stunden. Wegen zunächst nicht ausgeschlossener Interaktionen mit Zovirax (wegen Herpes Zoster) wurde die α-Liponsäure-Infusion während der Zovirax-Gabe unterbrochen (3 mal je 1-2 Stunden/Tag).

Es wurde der Plasma p24 Antigenspiegel bestimmt und Virusisolierungen durchgeführt.
Der Plasma p24 Antigenspiegel bewegte sich die Monate vor Therapiebeginn in einem Bereich von 0,350 ng/ml. 14 Tage nach Therapiebeginn sank dieser Wert auf 0,05 ng/ml, fiel dann bis zum 29. Tag noch weiter ab (auf 0,025 ng/ml ) und stieg dann auf einen Wert von 0,25 ng/ml bis zum Ende der Therapie wieder an. Die Virusisolierung (Standardmethode) war während der gesamten Therapie positiv.

### Patient 2 (Ro., 50 kg; Walter-Reed-Stadium 6)

Die Therapie-Phase dauerte 21 Tage, wobei an insgesamt nur 17 Tagen das Medikament verabreicht wurde. Die Gesamtdosis betrug 32 g. Die Dosierung betrug 1 g an 2 Tagen, 3 g an 8 Tagen, 4 g an 1 Tag.

Plasma p24 Antigenspiegelbestimmung, Virusisolierung mit Zelltitration und Plasmatitration wurden bei diesem Patienten durchgeführt.
Der Plasma p24 Antigenspiegel lag vor Behandlung bei konstant 0,4 ng/ml. Dieser Wert sank zunächst auf knapp das 10fache ab. Der Zelltiter lag vor Behandlung bei 1000, d.h. aus nur 10³ Zellen konnte Virus isoliert werden (Standardmethode). Im Verlauf der Therapie stieg dieser Wert um zwei Logarithmen-Stufen an, das heißt das Virus konnte nur noch aus 10⁵ Zellen angezüchtet werden. Vor Therapie lag der Plasmatiter bei 100. Dieser Wert fiel bereits kurz nach Therapiebeginn auf 0 ab.
Gegen Ende und nach Abschluß der Therapie kam es zu einem drastischen Abfall sämtlicher Virusmarker. Selbst die Virusisolierung wurde wiederholt negativ.

### Patient 3 (Pal.; Walter-Reed-Stadium 6)

Gesamtdosis an α-Liponsäure von 104 g an 26 Tagen während der 27tägigen Therapie-Phase. An 2 Tagen wurden 2 g/Tag, an 2 Tagen 3 g/Tag, an 1 Tag 4 g/Tag, an 2 Tagen 5 g/Tag, an 3 Tagen 6 g/Tag verabreicht. Nach 1 Tag Beurlaubung ohne Therapie 2 Tage 6 g/Tag, dann Dosisreduktion auf 1 g über 4 Tage wegen zunehmender Thrombopenie. Darauf 1 Tag 3 g, 6 Tage 6 g/Tag, dann wegen erneuter Thrombozytenabnahme (Daraprim-Nebenwirkung) Dosisreduktion auf 1 g über 3 Tage und 1 Tag 2 g/Tag. Danach planmäßige Beendigung der Therapie. An 11 Tagen insgesamt wurde die maximale Dosis von 6 g/Tag verabreicht.

Bei diesem Patienten wurde der Plasma p24 Antigenspiegel (immer negativ) gemessen, Virusisolierungen sowie Plasmatitrationen (immer negativ) durchgeführt.
Vor Beginn der Therapie lag die Produktion von p24 Antigen in pg/ml/200000 Zellen bei etwa 10. Dieser Wert verdoppelte sich am 5. Tag der Therapie, um dann bis zum 28. Tag kontinuierlich bis auf 0 abzufallen. Nach Therapieende stieg dieser Wert wieder langsam an.

### Patient 4 (Pag., 75 kg; Walter-Reed-Stadium 6)

Therapiephase 19 Tage. Gesamtdosis α-Liponsäure: 82,75 g. Therapie ab 22.05.1990 mit α-Liponsäure-Dauerinfusion über 20-24 Stunden/Tag. Dosen: 2 Tage 2 g, 9 Tage 4 g, 1 Tag 3 g, 1 Tag 2,75 g, 3 Tage 6 g, 1 Tag 7 g, 2 Tage 6 g. Verabreichung als 20-24 Stunden Dauerinfusion mit 1 Pause von 1/2 Tag einmalig.

Sämtliche Parameter (Plasma p24 Antigenspiegel), Virusisolierung mit Zelltitration (Plasmatitration) waren bei diesem Patienten positiv und wurden im Verlauf der Therapie gemessen.
Der Plasma p24 Antigenspiegel, der sich kurz vor und zu Beginn der Therapie bei Werten um 0,070 ng/ml bewegte, fiel bis zum 18. Therapietag auf 0,005 ng/ml ab. Nach Therapieende stieg der Spiegel wieder auf die anfänglichen Werte an.
p24 Produktion/ml/200000 Zellen lag kurz vor Therapie bei 8 pg/ml. Bis zum Ende der Therapie fiel dieser Wert um 75 % ab und stieg auch nach Therapieende nicht wesentlich an. Zu diesem Zeitpunkt stand der Patient zusätzlich unter AZT Therapie. Der Plasmatiter, der vor Therapiebeginn bei 10 lag, fiel während der Therapie auf 0 ab, pendelte sich dann am Ende der Therapie und danach bei 1 ein. Vor Therapiebeginn war aus 2 x 10⁶ Zellen eindeutig Virus nachweisbar. Am Ende der Therapie waren Viren nur noch kaum (im gegenwärtigen Bereich) nachweisbar. Nach Beendigung der Therapie verschlechterten sich die Resultate wieder.

Zusammenfassend ergab sich folgendes Ergebnis:
Unter Thioctacid-Therapie fielen deutlich positive Plasma p24 Antigenspiegel (Fall 1 u. 2) innerhalb eines kurzen Zeitraumes (ca. 14 Tage) auf mindestens 1/10 ihres Ausgangswertes ab.
Bei den Virusisolierungen aus Patientenlymphozyten konnte in drei Fällen (Fall 2, 3 u. 4) eine deutlich geringere Virusproduktion im Verlauf der Therapie nachgewiesen werden. Bei zwei Patienten (Fall 2 und 3) wurde das Resultat sogar gänzlich negativ. Positive Plasmatiter sanken ebenfalls im Verlauf der Therapie auf 0 ab. (Fall 2 u. 4).

Diese Ergebnisse zeigen, daß bei Patienten, die deutlich meßbare virologische Marker aufweisen, unter Thioctacid-Therapie eine ausgeprägte Verbesserung dieser Parameter auftritt und daher, bei einer längerdauernden Behandlung mit α-Liponsäure mit einer positiven Wirkung auf das Gesamtkrankheitsbild zu rechnen ist.

Die Wirkungsrichtung der erfindungsgemäßen Verbindungen I hinsichtlich ihrer antiretroviralen Wirkung ist am ehesten mit der Wirkung des bekannten Arzneimittelwirkstoffes Immuthiol (Natrium-diethyl-dithiocarbonat) vergleichbar, jedoch wird im Unterschied hierzu beispielsweise der cytopathogene Effekt (CPE) durch die Wirkstoffe I wesentlich deutlicher unterdrückt, insbesondere wenn die Substanzen nach der Infektion der Zellen hinzugegeben werden.
Als Indikationen für Verbindungen I kommen hier zum Beispiel in Betracht: die therapeutische Behandlung HIV infizierter, asymtomatischer als auch symtomatisch erkrankter Personen aller Stadien des erworbenen Immundefizienz-Syndroms (AIDS) nach der international üblichen Klassifizierung. Es bestehen keine Kontraindikationen.

Dosierungsmengen und Anwendungsformen, die außer den bereits angegebenen insbesondere bei Verwendung zur Bekämpfung von Retroviren (insbesondere Aids) in Frage kommen:

Die pharmazeutischen Zubereitungen enthalten für diese Anwendung im allgemeinen zwischen 50 mg bis 3 g als Einzeldosis, vorzugsweise 100 mg bis 1 g insbesondere 400 mg oder 500 mg bis 1 g der erfindungsgemäßen aktiven Komponente der Formel I (vorzugsweise Thioctsäure oder Dihydroliponsäure). Die erreichten Wirkspiegel/kg Körpergewicht sollen zwischen 3,5 und 200 mg, vorzugsweise zwischen 7 und 100 mg, besonders zwischen 35 und 70 mg/kg Körpergewicht liegen. Der Wirkstoff I (das heißt Verbindung der Formel I) soll aus den Zubereitungen langsam abgegeben werden. Dies gilt auch für die Komponente b bei Kombinationen.

Die Verabreichung kann beispielsweise in Form von Tabletten, Kapseln, Pillen, Dragees, Aerosolen oder in flüssiger Form erfolgen.

Als flüssige Anwendungsformen kommen zum Beispiel in Frage: alkoholische beziehungsweise wässrige Lösungen sowie Suspensionen und Emulsionen.

Bevorzugte Anwendungsformen sind zum Beispiel Tabletten, die zwischen 100 mg und 2 g oder Lösungen, die zwischen 10 mg bis 0,2 g/ml Flüssigkeit an aktiver Substanz enthalten.

Die Einzeldosis an Wirstoff der Formel I kann beispielsweise liegen:
a) bei oralen Arzneiformen zwischen 100 mg - 3 g, vorzugsweise 200 mg - 1 g, insbesondere 400 mg bis 1 g.
b) bei parenteralen Arzneiformen (zum Beispiel intravenös, intramuskulär) zwischen 100 mg - 12 g, vorzugsweise 200 mg - 6 g, insbesondere 500 mg bis 6 g.
c) bei Arzneiformen zur Inhalation (Lösungen oder Aerosole) zwischen 100 mg - 2 g, vorzugsweise 200 mg -1 g.

Die Dosen gemäß a) bis c) können beispielsweise 1-bis 6mal, vorzugsweise 1-bis 4mal täglich oder aber als Dauerinfusion, beispielsweise mit Hilfe eines Infusionaten* verabreicht werden.
*) Infusionsgerät zur genauen stündlichen Dosierung einer gelösten Wirksubstanz

Die tägliche Dosis an Wirstoff der Formel I beim Menschen liegt zum Beispiel für die parenterale Applikation im Bereich von 40 - 80 mg, vorzugsweise 40 - 60 mg pro kg Gewicht (zum Beispiel intravenöse Dauerinfusion über 24 Stunden). Bei längerer Anwendung kann gegebenenfalls die Dosis gesteigert werden, beispielsweise bis zu 160 mg pro kg Gewicht innerhalb 24 Stunden. Bei oraler Applikation kann die tägliche Dosis beispielsweise zwischen 40 - 120 mg/kg Körpergewicht liegen (bei längerer Anwendung ebenfalls Erhöhungen bis 160 mg oder auch 200 mg pro kg Körpergewicht täglich); die Einzeldosis liegt zum Beispiel bei 16 - 20 mg pro kg Gewicht, wobei diese Dosis zweckmäßig 4mal verabreicht wird pro Tag. Vorzugsweise beträgt die Tagesdosis an Wirkstoff der Formel I 4 - 6 g: Die Arzneimittel enthalten daher vorzugsweise 1 - 1,5 g an Verbindung I in einer galenischen Formulierung, wobei eine solche Dosis vorzugsweise 4mal verabreicht wird.

Für die Behandlung können zum Beispiel 3 mal täglich 1 bis 4 Tabletten mit einem Gehalt von 50 mg bis 2 g, vorzugsweise 400 mg oder 500 mg bis 2 g wirksamer Substanz oder beispielsweise bei intravenösen Injektion 1 bis 4 mal täglich eine Ampulle/Infusionsflasche von 1 bis 500 ml Inhalt mit 200 mg bis 6 g insbesondere 500 mg bis 6 g Wirksubstanz empfohlen werden. Bei oraler Verabreichung ist die minimale tägliche Dosis beispielsweise 300 mg; die maximale tägliche Dosis bei oraler Verabreichung soll 12 g nicht überschreiten.
- Die angegebenen Dosismengen beziehen sich stets auf die freie Säuren der Formel I. Falls die Verbindungen I in Form ihrer Salze verwendet werden, sind die angegebenen Dosierungen/Dosierungsbereiche dem höheren Mol-Gewicht entsprechend zu erhöhen. -
Für die Kombination der Wirkstoffe der Formel I (zum Beispiel alpha-Liponsäure) mit der Komponente b, zum Beispiel AZT, können die beiden Komponenten jeweils zum Beispiel in einem Verhältnis von 1 zu 100 bis 100 zu 1 äquimolaren Anteilen wirksamer Substanz gemischt werden, insbesondere in einem Verhältnis von 1 zu 10 bis 10 zu 1, vorzugsweise in einem Verhältnis von 1 zu 3 bis 3 zu 1 Anteilen.
Im Falle einer Kombination von Wirkstoffen der Formel I (zum Beispiel α-Liponsäure) und α-Interferon können die beiden Komponenten zum Beispiel in folgendem Verhältnis vorliegen: 50 mg - 6 g Verbindung I (Komponente a) zu 8 x 10⁶ Enzymeinheiten bis 1 x 10⁵ Enzymeinheiten α-Interferon, insbesondere 0,5 - 3 g Komponente a zu 1-4 x 10⁶ Enzymeinheiten α-Interferon.

In der Kombination aus den Wirkstoffen I und anderen Komponenten gemäß b) können beide Komponenten als Gemisch vorliegen. Im allgemeinen liegen die Komponenten jedoch voneinander getrennt in einer galenischen Formulierung vor, wobei hier die hierfür bekannten galenischen Formulierungen in Frage kommen: zum Beispiel eine Komponente als Tablette oder lackierte Tablette, die andere Komponente als Pulver, beide in einer Kapsel und umgekehrt; eine Komponente in Form von Pellets, die andere als Pulver, Dragee oder Tablette und umgekehrt und wobei die beiden Formen zum Beispiel in einer Kapsel vorliegen; oder in Form von Mehrschichten- oder Manteltabletten. Es wird hierzu zum Beispiel auf das Buch von Karl Thoma, Arzneimittelstabilität, Frankfurt 1978, zum Beispiel Seite 207ff. verwiesen.

Die erfindungsgemäße Kombination kann aber auch als ein Erzeugnis vorliegen, bei dem jeweils die beiden Einzelwirkstoffe in vollständig voneinander getrennten Formulierungen vorliegen, wobei insbesondere die Komponente b, aber auch beide Komponenten (a und b) in Ampullen und/oder Infusionsflaschen enthalten sind, so daß auch eine getrennte oder auch zeitlich abgestufte Verabreichung möglich ist.

Falls solche vollständig getrennten Formulierungen vorliegen, sind diese aufeinander abgestimmt und enthalten die jeweiligen Wirkstoffe in der Dosierungseinheit in denselben Mengen und entsprechenden Gewichtsverhältnissen, in denen sie in der kombinierten Mischung vorliegen können.

Bei einem Erzeugnis für die getrennte Anwendung ist es auch möglich, daß beide Kombinationspartner nicht gleichzeitig verabreicht werden. In solchen Fällen kann zum Beispiel der Wirkstoff I als Dauerinfusion gegeben werden (Dosis zum Beispiel 2 - 5 g pro Tag) und die andere Komponente b gleichzeitig (Dosis zum Beispiel 50 - 800 mg oder 1-8 x 10⁶ Enzymeinheiten, vorzugsweise intramuskulär) oder auch als Dauerinfusion pro Tag oder der Wirkstoff I kann zum Beispiel 4mal pro Tag gegeben werden (Einzeldosis zum Beispiel 0,5 -2 g) und die andere Komponente b gleichzeitig (Dosis zum Beispiel 50 - 200 mg beziehungsweise 0,5 - 3 x 10⁶ Enzymeinheiten). Es können dann zum Beispiel 1 bis 3 weitere Dosen an Komponente b (zum Beispiel zwischen 50 - 200 mg beziehungsweise 0,5 -3 x 10⁶ Enzymeinheiten) im Abstand von jeweils 6 und/oder 12 Stunden folgen.

Die erfindungsgemäßen Zubereitungen/Erzeugnisse können vorzugsweise auch zusätzliche Vitamine enthalten, insbesondere Vitamin B₁ und/oder Vitamin E.

Die akute Toxizität der alpha-Liponsäure an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation über 400 mg/kg Körpergewicht, diejenige der Dihydroliponsäure oberhalb 200 mg/kg Körpergewicht Maus.

### Beispiele

### Beispiel 1:

### Tabletten mit 50 mg S- bzw. R-α-Liponsäure

250 g S-α-Liponsäure werden mit 750 g mikrokristalliner Cellulose gleichmäßig verrieben. Nach dem Sieben der Mischung werden 250 g Stärke (Starch 1500/ Colorcon), 732,5 g Lactose, 15 g Magnesiumstearat und 2,5 g hochdisperses Siliciumdioxid zugemischt und die Mischung zu Tabletten vom Gewicht 400,0 mg verpreßt.

Eine Tablette enthält 50 mg S-α-Liponsäure.

In gleicher Weise können Tabletten mit 50 mg R-α-Liponsäure hergestellt werden, wenn statt 250 g S-α-Liponsäure die gleiche Menge R-α-Liponsäure eingesetzt wird.

Gegebenenfalls können die Tabletten nach üblichen Verfahren mit einem magensaftlöslichen oder magensaftpermeablen Filmüberzug versehen werden.

### Beispiel 2:

### Ampullen mit 50 mg S- bzw. R-α-Liponsäure als Trometamolsalz in 2 ml

250 g S-α-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 10 Liter aufgefüllt und anschließend durch ein Membranfilter der Porenweite 0,2 »m mit Glasfaservorfilter filtriert. Das Filtrat wird unter aseptischen Bedingungen zu 2 ml in sterilisierte 2 ml-Ampullen abgefüllt.

Eine Ampulle enthält in 2 ml Injektionslösung 50 mg S-α-Liponsäure als Trometamolsalz.

In gleicher Weise können Ampullen mit R-α-Liponsäure hergestellt werden, wenn statt 250 g S-α-Liponsäure die gleiche Menge R-α-Liponsäure eingesetzt wird.

### Beispiel 3

### Ampullen mit 250 mg Dihydroliponsäure in 10 ml Injektionslösung

60 g Trometamol und 1 g Ethylendiamintetraessigsäure, Dinatriumsalz werden in 1,8 Liter Wasser für Injektionszwecke gelöst. Die Lösung wird 30 Minuten lang mit Stickstoff begast. Unter weiterer Begasung mit Stickstoff werden in der Mischung 2 g Natriumdisulfit und anschließend 50 g Dihydroliponsäure gelöst. Die Lösung wird mit Wasser für Injektionszwecke, das mit Stickstoff begast wurde, auf ein Volumen von 2 Liter aufgefüllt. Nach sorgfältigem Mischen wird die Lösung über ein Membranfilter der Porenweite 0,2 »m filtriert und das Filtrat unter aseptischen Bedingungen und unter Vor- und Nachbegasung mit Stickstoff in Ampullen zu 10 ml Füllvolumen abgefüllt.

Eine Ampulle enthält in 10 ml Lösung 250 mg Dihydroliponsäure als Trometamolsalz.

## Patentansprüche

1. Verwendung von Verbindungen der Formel 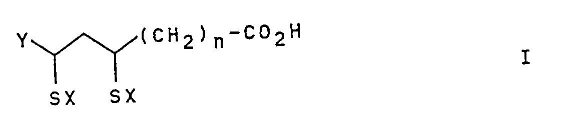 worin X ein Wasserstoffatom ist oder beide X eine einfache Bindung zwischen den beiden Schwefelatomen bedeuten; Y Wasserstoff oder C₁-C₆-Alkyl ist und n eine Zahl von 1 bis 10 darstellt oder deren therapeutisch verwertbaren Salzen für die Herstellung eines Arzneimittels zur Bekämpfung von Erkrankungen durch Retroviren.

2. Erzeugnis zur Bekämpfung von Retroviren, dadurch , gekennzeichnet, daß es neben den üblichen pharmazeutischen Träger- und/oder Verdünnungs-, beziehungsweise Hilfsstoffen
a) eine Verbindung der Formel 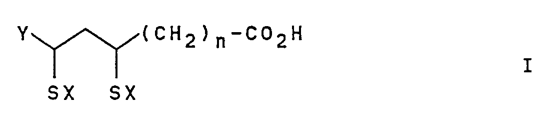 worin X ein Wasserstoffatom ist oder beide X eine einfache Bindung zwischen den beiden Schwefelatomen bedeuten; Y Wasserstoff oder C₁-C₆-Alkyl ist und n eine Zahl von 1 bis 10 darstellt und
b) eine andere antiretroviralwirksame Substanz oder jeweils ein physiologisch unbedenkliches Salz beider Wirkstoffe, in einer eine synergistische Wirkung erzeugenden Menge enthält und in einer Form vorliegt, die sowohl eine gemeinsame als auch eine getrennte therapeutische Anwendung beider Wirkstoffe gestattet.

3. Erzeugnis nach Anspruch 2,
dadurch gekennzeichnet, daß
die andere gegen Retroviren wirksame Substanz Azidothymidin (AZT) Didesoxyinosin (DDI), α-Interferon oder Didesoxycytidin (DDC) ist.

4. Erzeugnis nach Anspruch 2 oder 3,
dadurch gekennzeichnet, daß in der Kombination auf ein Gewichtsteil einer Verbindung der Formel I (Wirkkomponente I beziehungsweise Komponente a) jeweils 0,01 bis 100 Gewichtsteile der anderen Komponente gemäß b) oder auf 1 mg Wirkkomponente I (Komponente a) jeweils 20 - 200 000 Ezymeinheiten der anderen Komponente gemäß b) kommen.

5. Erzeugnis nach einem oder mehreren der Ansprüche 2-4, dadurch gekennzeichnet, daß
in der Dosiereinheit die Kombination 50 mg bis 6 g, vorzugsweise 200 mg bis 2 g Wirkkomponente I und 10 bis 300 mg, vorzugsweise 50 bis 200 mg beziehungsweise 5 x 10⁵ Enzymeinheiten (Units) bis 8 x 10⁶ Enzymeinheiten, vorzugsweise 1 x 10⁶ bis 4 x 10⁶ Enzymeinheiten der anderen Komponente gemäß b) enthält.

6. Mittel/Erzeugnis nach einem oder mehreren der vorangegangenen Ansprüche,
dadurch gekennzeichnet, daß
es zusätzliche Vitamine, vorzugsweise Vitamine B₁ und/oder E enthält.

7. Verfahren zur Herstellung eines Erzeugnisses nach einem oder mehreren der Ansprüche 2-6,
dadurch gekennzeichnet, daß
man 1 Gewichtsteil Wirkkomponente I und 0,01 bis 100 Gewichtsteile beziehungsweise 20 bis 200 000 Enzymeinheiten der anderen Komponente gemäß b), wobei die Wirkstoffe auch in Form ihrer Salze vorliegen können, zusammen mit üblichen Träger- und/oder Verdünnungs- beziehungsweise Hilfsstoffen sowie gegebenenfalls unter Zusatz von Vitaminen zu Erzeugnissen verarbeitet, welche in der Dosierungseinheit 50 mg bis 6 g Wirkkomponente I und 10 bis 300 mg beziehungsweise 5 x 10⁵ bis 8 x 10⁶ Enzymeinheiten der anderen Komponente gemäß b) enthalten.

## Claims

1. Use of compounds of the formula 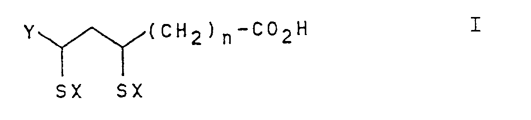 where X is a hydrogen atom or the two X's denote a single bond between the two sulphur atoms; Y is hydrogen or C₁-C₆ alkyl and n represents a number from 1 to 10 or their therapeutically usable salts for the production of a pharmaceutical preparation for the combating of diseases due to retroviruses.

2. Product for the combating of retroviruses, characterized in that it contains, in addition to the customary pharmaceutical carriers and/or diluents or adjuvants
a) a compound of the formula 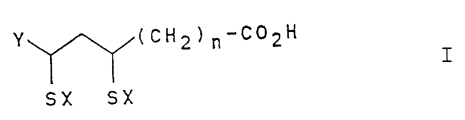 where X is a hydrogen atom or the two X's denote a single bond between the two sulphur atoms; Y is hydrogen or C₁-C₆ alkyl and n represents a number from 1 to 10 and
b) another substance with antiretroviral effect or a physiologically completely harmless salt of each of the two active substances in an amount producing a synergistic effect and is present in a form that permits both a joint and a separate therapeutic application of the two active substances.

3. Product according to Claim 2, characterized in that the other substance effective against retroviruses is azidothymidine (AZT), dideoxyinosine (DDI), α-interferon or dideoxycytidine (ddCyd).

4. Product according to Claim 2 or 3, characterized in that in the combination, for each 1 part by weight of a compound of formula I (active component I or component a)) there are 0.01 to 100 parts by weight of the other component according to b) or for each 1 mg of active component I (component a) there are 20 - 200,000 enzyme units of the other component according to b).

5. Product according to one or more of Claims 2-4, characterized in that in the unit dose the combination contains 50 mg to 6 g, preferably 200 mg to 2 g, of active component I and 10 to 300 mg, preferably 50 to 200 mg, or 5 x 10⁵ enzyme units (units) to 8 x 10⁶ enzyme units, preferably 1 x 10⁶ to 4 x 10⁶ enzyme units of the other component according to b).

6. Remedy/product according to one or more of the preceding claims, characterized in that it contains additional vitamins, preferably vitamin B₁ and/or E.

7. Process for the production of a product according to one or more of Claims 2-6, characterized in that 1 part by weight of active component I and 0.01 to 100 parts by weight or 20 to 200,000 enzyme units of the other component according to b), wherein the active substances can also be present in the form of their salts, together with customary carriers and/or diluents or adjuvants as well as optionally with the addition of vitamins are processed to products which in the unit dose contain 50 mg to 6 g of active component I and 10 to 300 mg or 5 x 10⁵ to 8 x 10⁶ enzyme units of the other component according to b).

## Revendications

1. Utilisation des composés de formule : 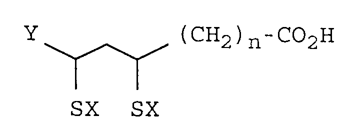 dans laquelle X est un atome d'hydrogène ou les deux X signifient une liaison simple entre les deux atomes de soufre.Y est un hydrogène ou un alcoyle en C₁-C₆ et n représente un nombre allant de 1 à 10 ou de leurs sels utilisables thérapeutiquement en vue de l'obtention d'un médicament pour la lutte contre les maladies dues à des rétrovirus.

2. Produit pour la lutte contre les rétrovirus, caractérisé en ce qu'il contient à côté des substance usuelles pharmaceutiques de support et/ou de dilution ou adjuvantes :
a) un composé de formule : 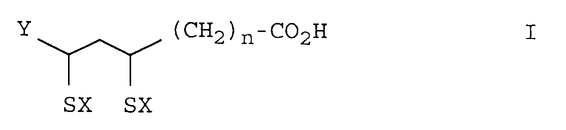 dans laquelle X est un atome d'hydrogène ou les deux X signifient une liaison simple entre les deux atomes de soufre. Y est de l'hydrogène ou un radical alcoyle en C₁-C₆ et n représente un nombre allant de 1 à 10, et
b) une autre substance active antirétrovirale ou respectivement un sel physiologiquement inoffensif de deux principes actifs en une quantité produisant une action synergique et qui se présente sous une forme qui permet aussi bien une utilisation conjointe qu'une utilisation thérapeutique séparée des deux principes actifs.

3. Produit selon la revendication 2, caractérisé en ce que l'autre substance active contre les rétrovirus est l'azidothymidine (AZT), la didesoxyinosine (DDI), l'α-interféron ou la didesoxycytidine (DDC).

4. Produit selon la revendication 2 ou 3, caractérisé en ce que dans la combinaison pour une partie en poids d'un composé de formule I (composant actif I ou composant a) respectivement de 0,01 à 100 parties en poids de l'autre composant selon b) ou pour 1 mg de composant actif I (composant a) viennent respectivement 20 à 200 000 unités d'enzyme de l'autre composant selon b).

5. Produit selon l'une ou plusieurs des revendications 2 à 4, caractérisé en ce que dans l'unité de dosage, la combinaison renferme de 50 mg à 6 g, de préférence de 200 mg à 2 g de composant actif I et de 10 à 300 mg, de préférence de 50 à 200 g ou de 5 x 10⁵ unités d'enzyme (units) à 8 x 10⁶ unités d'enzymes, de préférence de 1 x 10⁶ à 4 x 10⁶ unités d'enzyme, de l'autre composant selon b).

6. Agent/produit selon l'une ou plusieurs des revendications précédentes, caractérisé en ce qu'il renferme des vitamines additionnelles, de préférence de la vitamine B1 et/ou E.

7. Procédé d'obtention d'un produit selon l'une ou plusieurs des revendications 2 à 6, caractérisé en ce que l'on transforme 1 partie en poids de composant actif I et 0,01 à 100 parties en poids ou 20 à 200 000 unités d'enzyme de l'autre composant selon b), dans lesquels les principes actifs peuvent se présenter aussi sous la forme de leurs sels, conjointement avec des substances habituelles de support, et/ou de dilution ou auxiliaires ainsi que le cas échéant en ajoutant des vitamines aux produits, qui dans l'unité de dosage renferment de 50 mg à 6 g de composant actif I et de 10 à 300 mg, ou de 5 x 10⁵ à 8 x 10⁶ unités d'enzyme de l'autre composant selon b).
